# EUROPEAN PATENT SPECIFICATION

(11) **EP 2 766 188 B1**
(45) Date of publication and mention of the grant of the patent: **04.01.2017**
(21) Application number: 12840595.8
(22) Date of filing: 12.10.2012
(51) Int. Cl.: B41J 2/02, C12M 1/00

(54) **APPARATUS AND METHOD FOR USING ULTRASONIC RADIATION FOR CONTROLLED FRAGMENTATION OF CHAINS OF NUCLEIC ACIDS**
VORRICHTUNG UND VERFAHREN ZUR VERWENDUNG VON ULTRASCHALLSTRAHLUNG ZUR KONTROLLIERTEN FRAGMENTIERUNG VON NUKLEINSÄUREKETTEN
APPAREIL ET PROCÉDÉ POUR L'UTILISATION D'UN RAYONNEMENT ULTRASONORE POUR LA FRAGMENTATION RÉGULÉE DE CHAÎNES D'ACIDES NUCLÉIQUES

(30) Priority: 13.10.2011 US 201161546757 P
(43) Date of publication of application: 20.08.2014
(73) Proprietor: Microsonic Systems Inc., San Jose, CA 95134 (US)
(72) Inventor: VIVEK, Vibhu, Santa Clara, California 95050 (US); HADIMIOGLU, Babur, S-26233 Angelholm (SE); SHARMA, Smriti, Sunnyvale, California 94085 (US); DEV, Kapil, Providence, Rhode Island 02912 (US)
(74) Representative: Müller-Boré & Partner Patentanwälte PartG mbB
(86) International application number: PCT/US2012/059971
(87) International publication number: WO 2013/056062

(56) References cited:
- WO-A1-2009/124290
- WO-A2-2008/016691
- US-A- 5 890 802
- US-A1- 2004 119 793
- US-A1- 2009 254 289
- US-B1- 6 682 214
- LARGUINHO M ET AL: "Development of a fast and efficient ultrasonic-based strategy for DNA fragmentation", TALANTA, ELSEVIER, AMSTERDAM, NL, vol. 81, no. 3, 15 May 2010 (2010-05-15), pages 881-886, XP026966390, ISSN: 0039-9140 [retrieved on 2010-01-25]
- MANN T L ET AL: "The application of ultrasound as a rapid method to provide DNA fragments suitable for detection by DNA biosensors", BIOSENSORS AND BIOELECTRONICS, ELSEVIER BV, NL, vol. 20, no. 5, 15 November 2004 (2004-11-15), pages 945-955, XP004629279, ISSN: 0956-5663, DOI: 10.1016/J.BIOS.2004.06.021
- KAPIL DEV ET AL: "Design of a scalable DNA shearing system using phased-array ultrasonic transducer", ELECTRONICS, CIRCUITS AND SYSTEMS (ICECS), 2011 18TH IEEE INTERNATIONAL CONFERENCE ON, IEEE, 11 December 2011 (2011-12-11), pages 129-132, XP032095000, DOI: 10.1109/ICECS.2011.6122231 ISBN: 978-1-4577-1845-8

## Description

### FIELD

The present disclosure relates generally to ultrasonic devices and more particularly to ultrasonic devices for processing genetic material.

### BACKGROUND

Genomic research, including the study of nucleic acids such as DNA (Deoxyribonucleic acid) and RNA (Ribonucleic acid), has become increasingly important for life sciences, since it is well established that the complete information for the development and functioning of a living organism is encoded in its DNA. Therefore, there is a growing demand for reading the genomic sequence (e.g., DNA sequence) of humans and other organisms.

In order to perform sequencing operations accurately, conventional analysis tools (e.g., sequence readers) typically require that the initially long chains of nucleic acid be reduced to smaller chains. For example, the initial genomic sample may include chains with ten thousand or more base pairs or even billions of base pairs, and the operational setting for the sequence reader may require that the average length for the base-pair chains is less than five thousand (e.g., 300, 400, 600, 800, or 1,500 base pairs). Conventional approaches to fragmenting (or shearing) long chains, including enzymatic digestion, nebulization, hydroshear, and sonication, may also impose additional restrictions and disadvantages, including sample size requirements, sample loss, potential contamination, operational cost, and limited control. Thus, there is a need for improved systems and related methods for fragmenting genetic samples that include chains of nucleic acid.

An article by LARGUINHO M ET AL, "Development of a fast and efficient ultrasonic-based strategy for DNA fragmentation", TALANTA, ELSEVIER, AMSTERDAM, NL, 20100515, vol. 81, no. 3, Pages 881 - 886 4-14 discloses a comparative study of the use of ultrasonic devices for the fragmentation of DNA.

WO2008/016691 discloses a method and apparatus for the fragmentation of DNA using focused acoustic energy.

An article by MANN T L ET AL, "The application of ultrasound as a rapid method to provide DNA fragments suitable for detection by DNA biosensors", BIOSENSORS AND BIOELECTRONICS, ELSEVIER BV, NL, 20041115, vol. 20, no. 5, doi:10.1016/J.BIOS.2004.06.021, ISSN 0956-5663, PAGE 945 - 955 discloses the application of ultrasound for the fragmentation of DNA.

### SUMMARY

The invention provides a method and apparatus for using ultrasonic radiation for controlling fragmentation of a genetic sample as defined in the appended claims.

### BRIEF DESCRIPTION OF DRAWINGS

Some embodiments are illustrated by way of example and not limitation in the figures of the accompanying drawings.
Figure 1 is a diagram that shows a side view of a transducer system for an example embodiment.
Figure 2 is a diagram that shows a top view of a Fresnel Annular Sector Actuator (FASA) transducer for an example embodiment.
Figures 3A, 3B, and 3C are diagrams that show simulated acoustic displacements in cylindrical coordinates for the FASA transducer of Figure 2.
Figure 4 is a diagram that shows a top view of a composite FASA transducer for an example embodiment.
Figures 5A, 5B, and 5C are diagrams that show simulated acoustic displacements in cylindrical coordinates for the composite FASA transducer of Figure 4.
Figure 6 is a diagram that shows the distribution of fragments as a function of base-pair length for an experiment related to the embodiments of Figures 1 and 4.
Figure 7 is a diagram that shows a plot of the peak shear length for a given genetic sample size in an experiment related to the embodiments of Figures 1 and 4.
Figure 8 is a diagram that shows a plot of the mean length of DNA as a function of process time for two different genetic sample sizes in an experiment related to the embodiments of Figures 1 and 4.
Figure 9 is a diagram that shows a plot of the peak shear length for a given genetic sample size and two voltage amplitudes in an experiment related to the embodiments of Figures 1 and 4.
Figure 10 is a flowchart that shows a method of using ultrasonic radiation for controlled fragmentation of a genetic sample for an example embodiment.
Figure 11 is a diagram that shows a transducer system that includes an array of FASA transducer elements for an example embodiment.
Figure 12 is a flowchart that shows a method of using ultrasonic radiation for controlled fragmentation of a genetic sample for an example embodiment.
Figure 13 is a diagram that shows a computer-implemented transducer system for an example embodiment.
Figure 14 is a block diagram that shows a computer processing system within which a set of instructions for causing the computer to perform any one of the methodologies discussed herein may be executed.

### DETAILED DESCRIPTION

Example methods and systems are directed to controlled fragmentation of genetic samples that include chains of nucleic acid. The disclosed examples merely typify possible variations. Unless explicitly stated otherwise, components and functions are optional and may be combined or subdivided, and operations may vary in sequence or be combined or subdivided. In the following description, for purposes of explanation, numerous specific details are set forth to provide a thorough understanding of example embodiments. It will be evident to one skilled in the art, however, that the present subject matter may be practiced without these specific details.

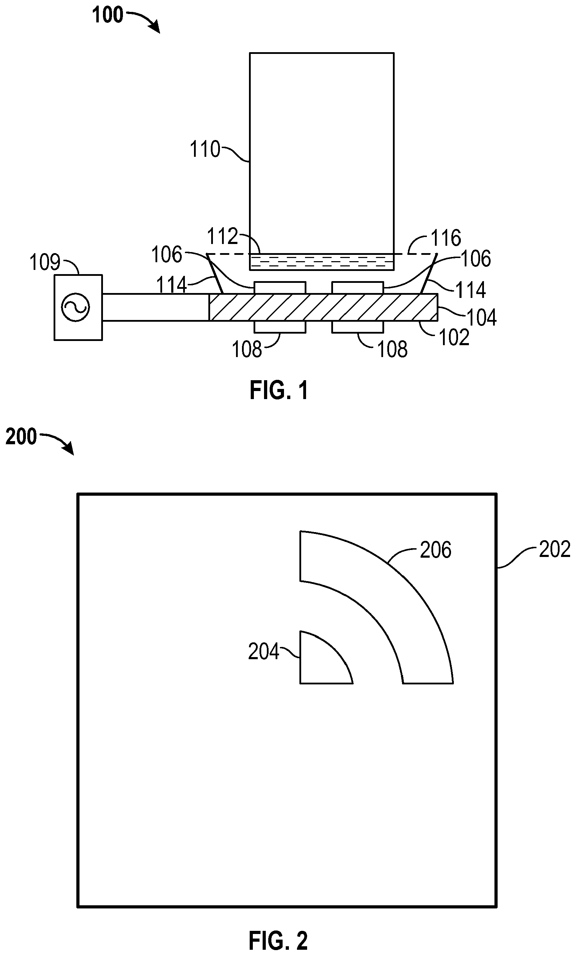

Figure 1 is a diagram that shows a side view of a system 100 that includes a transducer 102 and a sample container 110 for an example embodiment. The transducer 102 includes a substrate material 104 that is sandwiched between a top electrode 106 and a bottom electrode 108, where the substrate material 104 is typically a piezoelectric plate (e.g., made from lead zirconate titanate (PZT)), and the electrodes 106, 108 are electrically conducting material (e.g., metal). As discussed below, the electrodes 106, 108 are configured as a Fresnel Annular Sector Actuator (FASA) that directs acoustic energy in a target direction (e.g., upwards in Figure 1) when driven by a radio-frequency (RF) source 109 that is electrically connected to the electrodes 106, 108.

The sample container 110 contains a genetic sample 112 that includes chains of nucleic acid. For example, the genetic sample may include DNA, RNA, or lysed cells (e.g., cells that have been broken down by viral, enzymatic, or osmotic mechanisms or related mechanisms). Sidewalls 114 are disposed relative to the substrate material 104 to enclose a coupling medium 116 (e.g., a fluid such as water) that provides acoustic coupling between the substrate material 104 and the sample container 110. Additional details for the transducer system 100 may include a mechanical support (e.g., a support arm) that maintains the position of the sample container 110 relative to the transducer 102. Depending on the operational setting, an electrically insulating layer may be added to separate the top electrode 106 from the coupling medium 116.

Figure 2 is a diagram that shows a top view of a FASA transducer 200 that can be used as the transducer 102 of Figure 1. The top electrode of the FASA transducer 200 includes two annular segments 204, 206 that are disposed on a substrate 202 with a similar configuration on the bottom electrode. Figure 1 similarly shows two electrode elements for the top electrode 106 and the bottom electrode 108. The annular segments 204, 206 cover an angle of 90 degrees although other angles between 0 and 360 degrees are possible. Similarly, other configurations with increasing numbers of annular electrode elements are possible for FASA configurations. See, for example, U.S. Patent 6,682,214 ("Acoustic Wave Micromixer Using Fresnel Annular Sector Actuators"), which is incorporated herein by reference in its entirety.

In Figure 2 the substrate 202 has nominal dimensions of 8 mm x 8mm, and the width of each of the annular segments 204, 206 is about 1 mm. The elements in Figure 1 are not drawn to scale. In one example configuration the substrate material 104 is 8 mm x 8 mm as in Figure 2 with a thickness of 0.5 mm and the electrodes 106, 108 each have a width of about 1mm as in Figure 2 with a thickness that may vary from 0.1 micron to 50 microns depending on the details of the implementation. The sample container 110 may be a cylindrical tube with a diameter of about 6 mm and a height of about 4 cm, and the separation between the transducer 102 and the sample container 110 may be about 7mm. The genetic sample 112 (e.g., DNA solution) may be about 50 µL and may cover a length of about 3 mm at the bottom of the sample container 110. Although these dimensions are representative, alternative dimensions may be used depending on the operational setting.

Typically the RF source 109 in Figure 1 is driven with an electrical signal that is in the shape of a tone burst of a particular frequency that corresponds to thickness mode of resonance for the substrate material (e.g., PZT plate). That is, the frequency is chosen to be at or near a resonant mechanical frequency of the substrate to enhance the generation of acoustic waves (e.g., within 10%, 20%, or 30% of a fundamental frequency or higher harmonic). The shape of the drive waveform may be sinusoidal, square or any similar shape. The repetition rate is chosen to be sufficiently high to achieve the desired fragmentation relatively quickly without undesirably heating up the transducer or the genetic sample. Typical repetition rates range from tens of Hz to thousands of Hz. For example, in a typical configuration the resonant frequency is 4MHz (e.g., corresponding a PZT plate thickness of 0.5 mm), the drive waveform is applied for 50 cycles (∼12.µs), no signal is applied for 10⁴ cycles (∼2.5ms), and the corresponding duty cycle is D=50/10,050 = ∼0.5%. When the duration and amplitude of the tone burst are adjusted systematically, the mechanical forces exerted on the genetic sample 112 produce shearing forces that fragment the nucleic-acid chains into smaller chains in a controlled manner.

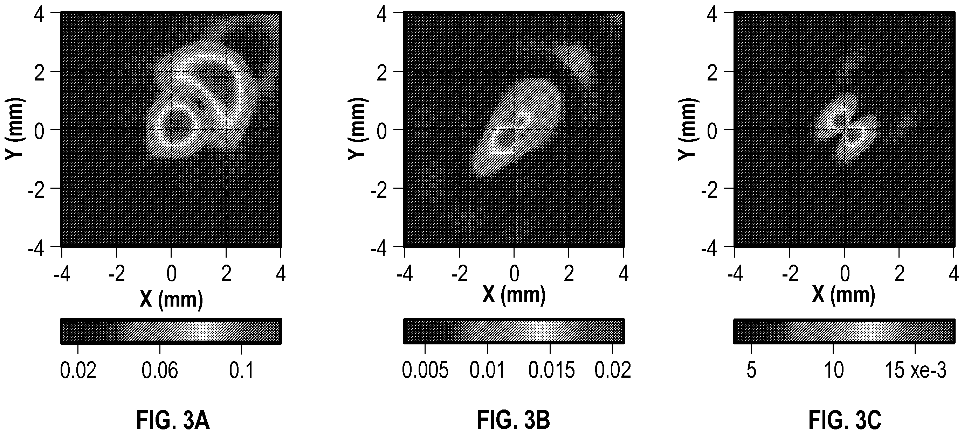

For example, Figures 3A, 3B, and 3C are diagrams that show simulated acoustic displacements in cylindrical coordinates for the FASA transducer 200 of Figure 2 at a nominal height that corresponds to the position of the genetic sample 112 in the sampler container 110 of Figure 1. Figure 3A shows the vertical component and Figures 3B and 3C respectively show the radial and angular components in the lateral direction (e.g., parallel to the surface of the substrate material 104).

Additional embodiments relate to alternative FASA configurations. For example FASA transducers of a suitable angle can with a small gap (e.g., a few microns to a few millimeters) between them to form a composite transducer.

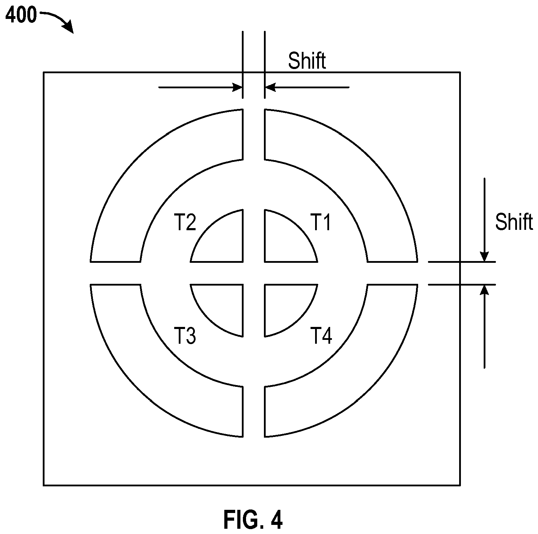

Additional embodiments relate to alternative FASA configurations. For example, a combination of FASA transducers, each with a suitable angle and a relatively small gap between them (e.g., a few microns to a few millimeters), can form a composite transducer that produces a higher level of mechanical energy to speed up the shearing process, while still resulting in a fair amount of lateral acoustic field in the coupling medium. Figure 4 shows a top view of a composite FASA transducer 400 that includes a composite electrode with four 90-degree elements labeled as transducers T1, T2, T3, and T4. The small gap, labeled as a shift between the elements, enables the composite transducer to effectively combine the mechanical effects of the individual transducers.

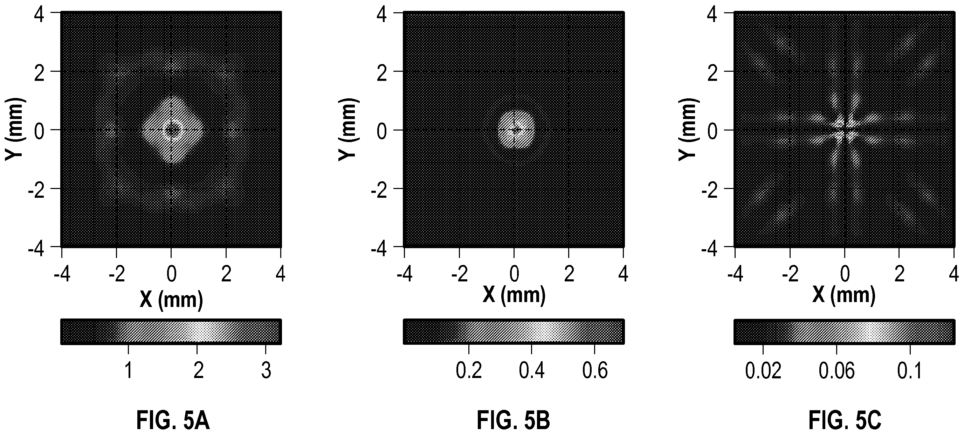

Similarly as in Figures 3A, 3B, and 3C, Figures 5A, 5B, and 5C are diagrams that show simulated acoustic displacements in cylindrical coordinates for the composite FASA transducer 400 of Figure 4 at a nominal height that corresponds to the position of the genetic sample 112 in the sample container 110 of Figure 1. Figure 5A shows the vertical component and Figures 5B and 5C respectively show the radial and angular components in the lateral direction (e.g., parallel to the surface of the substrate material 104). The constructive effect of the 90-degree elements is apparent from comparing the numerical indices in the magnitude scales in the Figures (e.g., 0.02 mm to 0.1 mm in Figure 3A versus 1 mm to 3 mm in Figure 5A).

By using a composite type of transducer such as the composite FASA transducer 400 in Figure 4, one can control the relative phase and/or amplitude of the RF signals applied to each element (e.g., each one of the four FASA elements T1-T4 in Figure 4) to control the shearing rate and/or steering the maximum acoustic intensity location within the sample container (i.e., similar to a "phased-array transducer"). That is, the phase combinations of the waveform inputs to the component elements of the FASA configuration may be used control the shearing process.

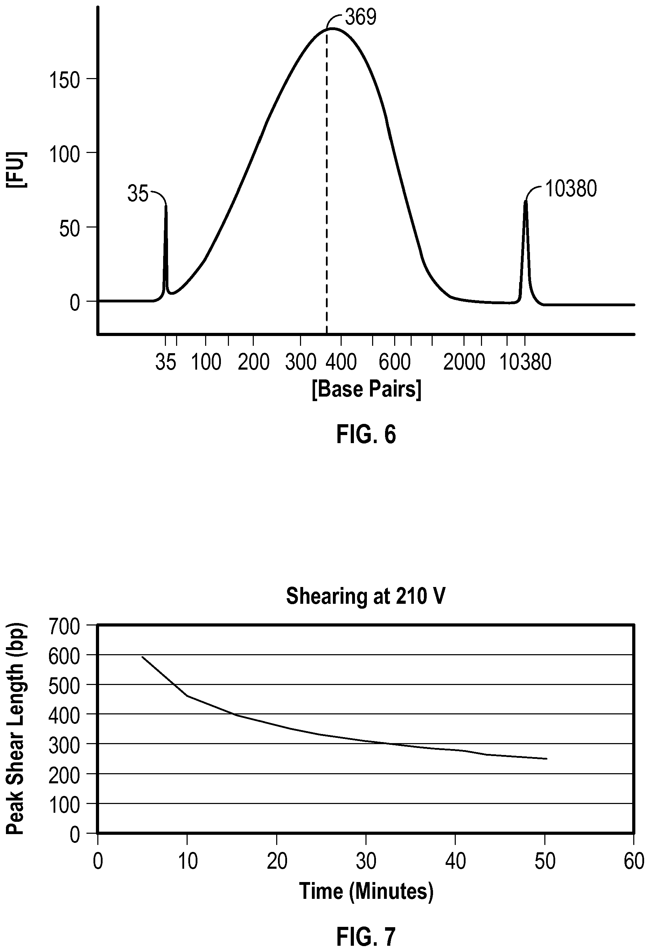

Figure 6 is a related diagram that shows the distribution of fragments (e.g., as measured in Fluorescence Units (FU)) as a function of base-pair length for an experiment of the FASA transducer 200 of Figure 2 over a specified time interval where the resulting fragment size has a mean value of 369 base pairs (BP). The values in Figure 6 correspond to a graph obtained from a gel electrophoresis image of a sheared sample and show the distribution of fragment lengths for a processed DNA sample. The results were obtained from an optical instrument that measures the sample's fluorescence, which is roughly proportional to the concentration of DNA at a particular length. As illustrated in Figure 6, the size distribution of the sheared DNA is relatively tight (e.g., small variance) so that most of the sheared DNA is relatively close to the mean length (369 BP). As discussed above, the shearing process can be controlled with the pulse parameters, such as the amplitude and duration of the pulses, as well as the total processing time. As result, the mean length of the sheared DNA can be similarly controlled by the pulse parameters.

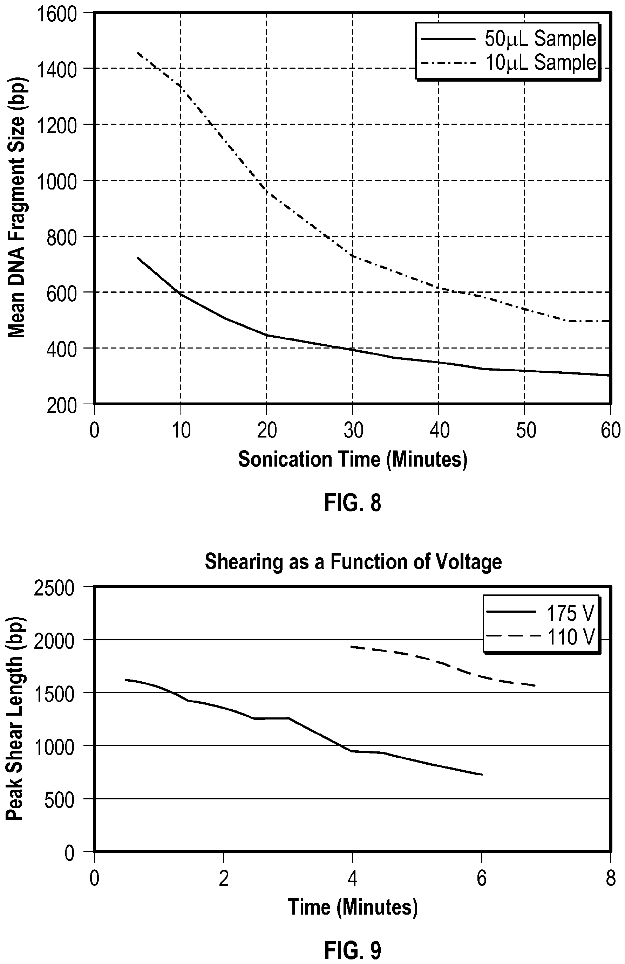

In particular, when the other parameters are set, the total processing time and the voltage amplitude are easily controllable and reproducible in a variety of operational settings. Figure 7 is a diagram that shows a plot of the peak shear length (e.g., maximal number of base pairs as in Figure 6) for a given genetic sample size (e.g., 50 µL). Figure 8 is a diagram that shows a plot of the mean length of DNA as a function of process time for two different genetic sample sizes: a 50 µL sample size and a 100 µL sample size. As illustrated in Figure 8, operations with a larger genetic sample size typically take more time to reach a target value for the mean fragment size. Figure 9 is a diagram that shows a plot of the peak shear length for a given genetic sample size (e.g., 50 µL) and two voltage amplitudes: 110 V and 175 V. As illustrated in Figure 9, operations with a lower voltage amplitude typically take more time to reach a target value for the mean fragment size.

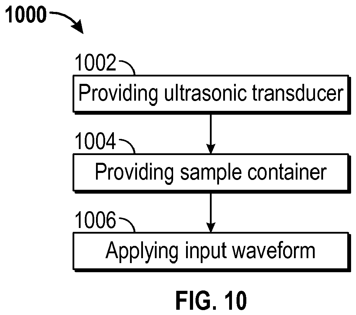

With reference to the system 100 of Figure 1, Figure 10 is a flowchart that shows a method 1000 of using ultrasonic radiation for controlled fragmentation of a genetic sample 112 that includes chains of nucleic acids according to an example embodiment. A first operation 1002 includes providing a transducer 102 that includes a substrate material 104 with top and bottom electrodes 106, 108 configured as a Fresnel Annular Sector Actuator (FASA) that directs acoustic energy in a target direction. A second operation 1004 includes providing a sample container 110 to contain the genetic sample 112, the sample container110 being disposed in the target direction of the transducer102, and the sample container 110 being acoustically coupled to the transducer 102 through a coupling medium 116. A third operation 1006 includes applying an input waveform to the transducer 102 to direct acoustic energy to the genetic sample 112 over a specified time interval, the genetic sample 112 being reduced to fragments of nucleic acid having an average fragment size that corresponds to the specified time interval.

Additional embodiments also relate to alternative FASA configurations that replace the annularly shaped electrode segments (e.g., as in Figure 2) with alternative electrode elements combined with Fresnel lenses that are disposed near the transducer plate. See, for example, U.S. Patent Application No. 12/418,503 ("Methods and Systems to Form High Efficiency and Uniform Fresnel Lens Arrays for Ultrasonic Liquid Manipulation"), which is incorporated herein by reference in its entirety.

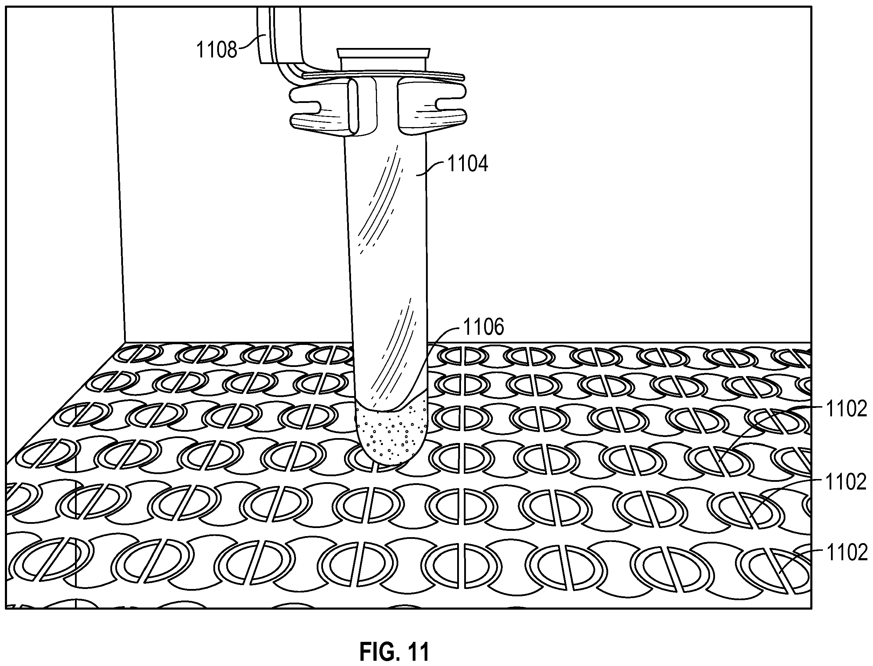

In some operational settings, it may be desirable to process multiple genetic samples simultaneously. Figure 11 is a diagram that shows a transducer system 1100 that includes an array of FASA transducer elements 1102. A sample container 1104 that contains a genomic sample 1106 is supported by a tube holder 1108. Although a single sample container 1104 is shown in Figure 11, multiple sample containers can be concurrently processed.

A computer system may be used to access a database that characterizes relationships between the parameters of the tone-burst signal as well as other configurations parameters that characterize the system including the position of the sample container 110 relative to the transducer 102 in Figure 1. As discussed above, the parameters of the tone-burst signal may include pulse amplitude, pulse duration, repetition rate, duty cycle, RF frequency, and phase combinations of the waveform inputs to the component elements of a FASA configuration (e.g., as in Figure 4). In some example embodiments, the specified time interval for processing the genetic sample 112 may be functionally related to one or more parameters of the tone-burst signal and a target fragment size for the average fragment size.

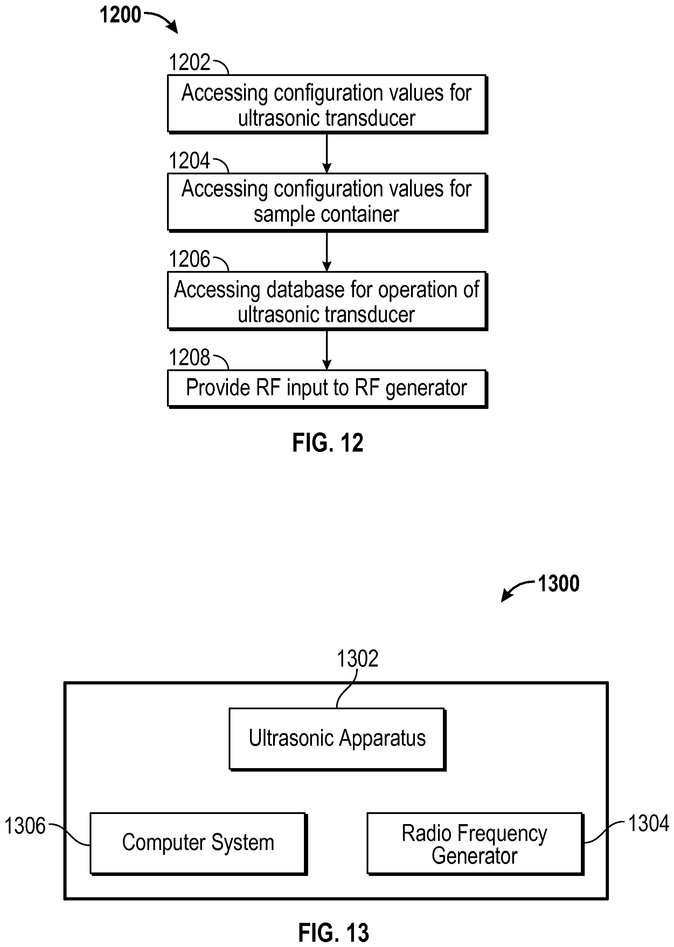

With reference to the transducer system 100 of Figure 1, Figure 12 is a flowchart that shows a method 1200 of using ultrasonic radiation for controlled fragmentation of a genetic sample 112 that includes chains of nucleic acids. A first operation 1202 includes accessing configuration values for transducer 102 that includes substrate material 104 with top and bottom electrodes 106, 108 configured as a Fresnel Annular Sector Actuator (FASA) that directs acoustic energy in a target direction. A second operation 1204 includes accessing configuration values for the sample container 110 that contains the genetic sample 112, the sample container 110 being disposed in the target direction of the transducer 102, and the sample container 110 being acoustically coupled to the transducer 102 through a coupling medium 116. A third operation 1206 includes accessing a database for operation of the transducer 102 with the sample container 110 under conditions given by the configuration values for the transducer 102 and the configuration values of the sample container 110, the database relating an application of an input waveform to the transducer 102 for a specified time interval to an average fragment size for fragments of nucleic acid in a controlled fragmentation of the genetic sample 112. A fourth operation 1208 includes providing an RF (radio-frequency) input to an RF generator (e.g., RF source 109) configured to apply the input waveform to the transducer 102.

Figure 13 is a diagram that shows a related computer-implemented transducer system 1300 including an ultrasonic apparatus 1302, a radio-frequency generator 1304, and a computer system 1306.

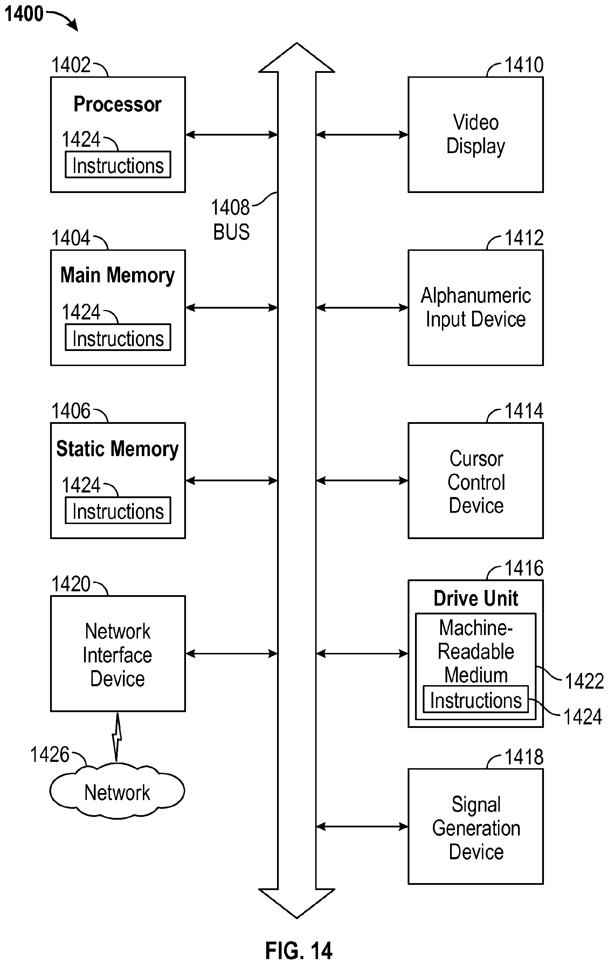

Figure 14 shows a machine in the example form of a computer system 1400 within which instructions for causing the machine to perform any one or more of the methodologies discussed here may be executed. In alternative embodiments, the machine operates as a standalone device or may be connected (e.g., networked) to other machines. In a networked deployment, the machine may operate in the capacity of a server or a client machine in server-client network environment, or as a peer machine in a peer-to-peer (or distributed) network environment. The machine may be a personal computer (PC), a tablet PC, a set-top box (STB), a personal digital assistant (PDA), a cellular telephone, a web appliance, a network router, switch or bridge, microcontroller, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein.

The example computer system 1400 includes a processor 1402 (e.g., a central processing unit (CPU), a graphics processing unit (GPU) or both), a main memory 1404, and a static memory 1406, which communicate with each other via a bus 1408. The computer system 1400 may further include a video display unit 1410 (e.g., a liquid crystal display (LCD) or a cathode ray tube (CRT)). The computer system 1400 also includes an alphanumeric input device 1412 (e.g., a keyboard), a user interface (UI) cursor control device 1414 (e.g., a mouse), a disk drive unit 1416, a signal generation device 1418 (e.g., a speaker), and a network interface device 1420.

In some contexts, a computer-readable medium may be described as a machine-readable medium. The disk drive unit 1416 includes a machine-readable medium 1422 on which is stored one or more sets of data structures and instructions 1424 (e.g., software) embodying or utilizing any one or more of the methodologies or functions described herein. The instructions 1424 may also reside, completely or at least partially, within the static memory 1406, within the main memory 1404, or within the processor 1402 during execution thereof by the computer system 1400, with the static memory 1406, the main memory 1404, and the processor 1402 also constituting machine-readable media.

While the machine-readable medium 1422 is shown in an example embodiment to be a single medium, the terms "machine-readable medium" and "computer-readable medium" may each refer to a single medium or multiple media (e.g., a centralized or distributed database, and/or associated caches and servers) that store the one or more sets of data structures and instructions 1424. These terms shall also be taken to include any tangible or non-transitory medium that is capable of storing, encoding or carrying instructions for execution by the machine and that cause the machine to perform any one or more of the methodologies disclosed herein, or that is capable of storing, encoding or carrying data structures utilized by or associated with such instructions. These terms shall accordingly be taken to include, but not be limited to, solid-state memories, optical media, and magnetic media. Specific examples of machine-readable or computer-readable media include non-volatile memory, including by way of example semiconductor memory devices, e.g., erasable programmable read-only memory (EPROM), electrically erasable programmable read-only memory (EEPROM), and flash memory devices; magnetic disks such as internal hard disks and removable disks; magneto-optical disks; compact disc read-only memory (CD-ROM) and digital versatile disc read-only memory (DVD-ROM).

The instructions 1424 may further be transmitted or received over a communications network 1426 using a transmission medium. The instructions 1424 may be transmitted using the network interface device 1420 and any one of a number of well-known transfer protocols (e.g., hypertext transfer protocol (HTTP)). Examples of communication networks include a local area network (LAN), a wide area network (WAN), the Internet, mobile telephone networks, plain old telephone (POTS) networks, and wireless data networks (e.g., WiFi and WiMax networks). The term "transmission medium" shall be taken to include any intangible medium that is capable of storing, encoding or carrying instructions for execution by the machine, and includes digital or analog communications signals or other intangible media to facilitate communication of such software.

Certain embodiments are described herein as including logic or a number of components, modules, or mechanisms. Modules may constitute either software modules or hardware-implemented modules. A hardware-implemented module is a tangible unit capable of performing certain operations and may be configured or arranged in a certain manner. In example embodiments, one or more computer systems (e.g., a standalone, client or server computer system) or one or more processors may be configured by software (e.g., an application or application portion) as a hardware-implemented module that operates to perform certain operations as described herein.

In various embodiments, a hardware-implemented module (e.g., a computer-implemented module) may be implemented mechanically or electronically. For example, a hardware-implemented module may comprise dedicated circuitry or logic that is permanently configured (e.g., as a special-purpose processor, such as a field programmable gate array (FPGA) or an application-specific integrated circuit (ASIC)) to perform certain operations. A hardware-implemented module may also comprise programmable logic or circuitry (e.g., as encompassed within a general-purpose processor or other programmable processor) that is temporarily configured by software to perform certain operations. It will be appreciated that the decision to implement a hardware-implemented module mechanically, in dedicated and permanently configured circuitry, or in temporarily configured circuitry (e.g., configured by software) may be driven by cost and time considerations.

Accordingly, the term "hardware-implemented module" (e.g., a "computer-implemented module") should be understood to encompass a tangible entity, be that an entity that is physically constructed, permanently configured (e.g., hardwired), or temporarily or transitorily configured (e.g., programmed) to operate in a certain manner and/or to perform certain operations described herein. Considering embodiments in which hardware-implemented modules are temporarily configured (e.g., programmed), each of the hardware-implemented modules need not be configured or instantiated at any one instance in time. For example, where the hardware-implemented modules comprise a general-purpose processor configured using software, the general-purpose processor may be configured as respective different hardware-implemented modules at different times. Software may accordingly configure a processor, for example, to constitute a particular hardware-implemented module at one instance of time and to constitute a different hardware-implemented module at a different instance of time.

Hardware-implemented modules can provide information to, and receive information from, other hardware-implemented modules. Accordingly, the described hardware-implemented modules may be regarded as being communicatively coupled. Where multiple of such hardware-implemented modules exist contemporaneously, communications may be achieved through signal transmission (e.g., over appropriate circuits and buses) that connect the hardware-implemented modules. In embodiments in which multiple hardware-implemented modules are configured or instantiated at different times, communications between such hardware-implemented modules may be achieved, for example, through the storage and retrieval of information in memory structures to which the multiple hardware-implemented modules have access. For example, one hardware-implemented module may perform an operation and store the output of that operation in a memory device to which it is communicatively coupled. A further hardware-implemented module may then, at a later time, access the memory device to retrieve and process the stored output. Hardware-implemented modules may also initiate communications with input or output devices and may operate on a resource (e.g., a collection of information).

The various operations of example methods described herein may be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example embodiments, comprise processor-implemented modules.

Similarly, the methods described herein may be at least partially processor-implemented. For example, at least some of the operations of a method may be performed by one or more processors or processor-implemented modules. The performance of certain of the operations may be distributed among the one or more processors, not only residing within a single machine, but deployed across a number of machines. In some example embodiments, the processor or processors may be located in a single location (e.g., within a home environment, an office environment or as a server farm), while in other embodiments the processors may be distributed across a number of locations.

The one or more processors may also operate to support performance of the relevant operations in a "cloud computing" environment or as a "software as a service" (SaaS). For example, at least some of the operations may be performed by a group of computers (as examples of machines including processors), these operations being accessible via a network (e.g., the Internet) and via one or more appropriate interfaces (e.g., application program interfaces (APIs)).

Although only certain embodiments have been described in detail above, those skilled in the art will readily appreciate that many modifications are possible without materially departing from the novel teachings of this disclosure. For example, aspects of embodiments disclosed above can be combined in other combinations to form additional embodiments. Accordingly, all such modifications are intended to be included within the scope of this disclosure.

## Claims

1. A method of using ultrasonic radiation for controlled fragmentation of a genetic sample that includes chains of nucleic acids, the method comprising:
providing a transducer (102) that includes a substrate (104) with top and bottom electrodes (106,108) configured as a Fresnel Annular Sector Actuator (FASA) that directs acoustic energy in a target direction;
providing a sample container (110) to contain the genetic sample, the sample container (110) being disposed in the target direction of the transducer (102), and the sample container (110) being acoustically coupled to the transducer (102) through a coupling medium (116); and
applying an input waveform to the transducer (102) to direct acoustic energy to the genetic sample over a specified time interval, the genetic sample being reduced to fragments of nucleic acid having an average fragment size that corresponds to the specified time interval, wherein the input waveform comprises tone bursts having a selected repetition rate and duty cycle.

2. The method of claim 1, wherein the substrate (104) includes piezoelectric material and the electrodes (106, 108) include electrically conductive material.

3. The method of claim 1, wherein the genetic sample includes at least one of DNA (Deoxyribonucleic acid), RNA (Ribonucleic acid), or lysed cells.

4. The method of claim 1, wherein the input waveform includes a frequency that is approximately a resonant frequency of the substrate (104).

5. The method of claim 1, wherein the specified time interval is correlated with a target fragment size that approximates the average fragment size when the input waveform is applied to the transducer (102) to direct acoustic energy to the genetic sample over the specified time interval.

6. The method of claim 1, further comprising:
estimating the specified time interval as a function of a voltage level for the input waveform and a target fragment size for the average fragment size; or
estimating the specified time interval as a function of a target fragment size for the average fragment size and the repetition rate of the tone bursts for the input waveform; or
estimating the specified time interval as a function of a target fragment size for the average fragment size and the duty cycle of the tone bursts for the input waveform; or
estimating the specified time interval as a function of a target fragment size for the average fragment size and an RF frequency of the input waveform; or
estimating the specified time interval as a function of a target fragment size for the average fragment size and a phase combination of the tone bursts for the input waveform at component elements of the FASA configuration.

7. The method of claim 1, wherein the top and bottom electrodes (106, 108) each include a plurality of annular segments that cover an angular sector between 0 and 360 degrees.

8. The method of claim 1, wherein the top and bottom electrodes (106, 108) each include a composite electrode that includes a plurality of sector elements that divide an area into approximately equal sectors, each sector element including a plurality of annular segments that each cover a corresponding angular sector.

9. The method of claim 1, wherein the tone bursts having a selected shape.

10. The method of claim 1, wherein a duration and amplitude of the tone bursts are adjusted systematically.

11. An apparatus for using ultrasonic radiation for controlled fragmentation of a genetic sample that includes chains of nucleic acids, the apparatus comprising:
a transducer (102) that includes a substrate (104) with top and bottom electrodes (106, 108) configured as a Fresnel Annular Sector Actuator (FASA) that directs acoustic energy in a target direction;
a sample container (110) configured to contain the genetic sample, the sample container (110) being disposed in the target direction of the transducer (102), and the sample container (110) being acoustically coupled to the transducer (102) through a coupling medium (116); and
a radio-frequency generator (109) configured to apply an input waveform to the transducer (102) to direct acoustic energy to the genetic sample over a specified time interval, the genetic sample being reduced to fragments of nucleic acid having an average fragment size that corresponds to the specified time interval, wherein the input waveform comprises tone bursts having a selected repetition rate and duty cycle.

12. The apparatus of claim 11, wherein the specified time interval is correlated with a target fragment size that approximates the average fragment size when the input waveform is applied to the transducer (102) to direct acoustic energy to the genetic sample over the specified time interval.

13. The apparatus of claim 11, wherein the radio-frequency generator (109) is further configured to:
estimate the specified time interval as a function of a voltage level for the input waveform and a target fragment size for the average fragment size; or
estimate the specified time interval as a function of a target fragment size for the average fragment size and the repetition rate of the tone bursts for the input waveform; or
estimate the specified time interval as a function of a target fragment size for the average fragment size and the duty cycle of the tone bursts for the input waveform; or
estimate the specified time interval as a function of a target fragment size for the average fragment size and an RF frequency of the input waveform; or
estimate the specified time interval as a function of a target fragment size for the average fragment size and a phase combination of tone bursts for the input waveform at component elements of the FASA configuration.

14. The apparatus of claim 11, wherein the radio-frequency generator (109) is further configured to use the tone bursts having a selected shape.

15. The apparatus of claim 11, wherein the radio-frequency generator (109) is further configured to adjust a duration and amplitude of the tone bursts systematically.

## Patentansprüche

1. Verfahren zur Verwendung von Ultraschallstrahlung zur kontrollierten Fragmentierung einer genetischen Probe, die Nukleinsäureketten enthält, wobei das Verfahren umfasst:
Bereitstellen eines Wandlers (102), der ein Substrat (104) mit oberen und unteren Elektroden (106, 108) umfasst, der als ein Fresnel-Ringsektoraktuator (FASA) konfiguriert ist, der akustische Energie in eine Zielrichtung leitet;
Bereitstellen eines Probenbehälters (110), um die genetische Probe zu enthalten, wobei der Probenbehälter (110) in der Zielrichtung des Wandlers (102) angeordnet ist und der Probenbehälter (110) durch ein Kopplungsmedium (116) akustisch mit dem Wandler (102) gekoppelt ist; und
Anlegen einer Eingangswellenform an den Wandler (102), um über ein spezifiziertes Zeitintervall akustische Energie zu der genetischen Probe zu leiten, wobei die genetische Probe auf Fragmente von Nukleinsäure mit einer mittleren Fragmentgröße reduziert wird, die dem spezifizierten Zeitintervall entspricht, wobei die Eingangswellenform Tonbursts mit ausgewählter Wiederholungsrate und relativer Einschaltdauer umfasst.

2. Verfahren nach Anspruch 1, wobei das Substrat (104) piezoelektrisches Material umfasst und die Elektroden (106, 108) elektrisch leitfähiges Material umfassen.

3. Verfahren nach Anspruch 1, wobei die genetische Probe DNA (Desoxyribonukleinsäure) und/oder RNA (Ribonukleinsäure) und/oder lysierte Zellen enthält.

4. Verfahren nach Anspruch 1, wobei die Eingangswellenform eine Frequenz umfasst, die ungefähr eine Resonanzfrequenz des Substrats (104) ist.

5. Verfahren nach Anspruch 1, wobei das spezifizierte Zeitintervall mit einer Zielfragmentgröße korreliert ist, die sich der mittleren Fragmentgröße nähert, wenn die Eingangswellenform an den Wandler (102) angelegt wird, um über das spezifizierte Zeitintervall akustische Energie zu der genetischen Probe zu leiten.

6. Verfahren nach Anspruch 1, das ferner umfasst:
Schätzen des spezifizierten Zeitintervalls als eine Funktion eines Spannungspegels für die Eingangswellenform und einer Zielfragmentgröße für die mittlere Fragmentgröße; oder
Schätzen des spezifizierten Zeitintervalls als eine Funktion einer Zielfragmentgröße für die mittlere Fragmentgröße und der Wiederholungsrate der Tonbursts für die Eingangswellenform; oder
Schätzen des spezifizierten Zeitintervalls als eine Funktion einer Zielfragmentgröße für die mittlere Fragmentgröße und der mittleren Einschaltdauer der Tonbursts für die Eingangswellenform; oder
Schätzen des spezifizierten Zeitintervalls als eine Funktion einer Zielfragmentgröße für die mittlere Fragmentgröße und einer RF-Frequenz der Eingangswellenform; oder
Schätzen des spezifizierten Zeitintervalls als eine Funktion einer Zielfragmentgröße für die mittlere Fragmentgröße und einer Phasenkombination der Tonbursts für die Eingangswellenform an Komponentenelementen der FASA-Konfiguration.

7. Verfahren nach Anspruch 1, wobei die oberen und unteren Elektroden (106, 108) jeweils eine Vielzahl ringförmiger Segmente umfassen, die einen Winkelsektor zwischen 0 und 360 Grad abdecken.

8. Verfahren nach Anspruch 1, wobei die oberen und unteren Elektroden (106, 108) jeweils eine zusammengesetzte Elektrode umfassen, die eine Vielzahl von Sektorelementen umfassen, die einen Bereich in ungefähr gleich große Sektoren unterteilen, wobei jedes Sektorelement eine Vielzahl ringförmiger Segmente umfasst, die einen entsprechenden winkelförmigen Sektor abdecken.

9. Verfahren nach Anspruch 1, wobei die Tonbursts eine ausgewählte Form haben.

10. Verfahren nach Anspruch 1, wobei eine Dauer und Amplitude der Tonbursts systematisch eingestellt werden.

11. Vorrichtung zur Verwendung von Ultraschallstrahlung zur kontrollierten Fragmentierung einer genetischen Probe, die Nukleinsäureketten enthält, wobei die Vorrichtung umfasst:
einen Wandler (102), der ein Substrat (104) mit oberen und unteren Elektroden (106, 108) umfasst, der als ein Fresnel-Ringsektoraktuator (FASA) konfiguriert ist, der akustische Energie in eine Zielrichtung leitet;
einen Probenbehälter (110), der konfiguriert ist, um die genetische Probe zu enthalten, wobei der Probenbehälter (110) in der Zielrichtung des Wandlers (102) angeordnet ist und der Probenbehälter (110) durch ein Kopplungsmedium (116) akustisch mit dem Wandler (102) gekoppelt ist; und
einen Funkfrequenzgenerator (109), der konfiguriert ist, um eine Eingangswellenform an den Wandler (102) anzulegen, um über ein spezifiziertes Zeitintervall akustische Energie zu der genetischen Probe zu leiten, wobei die genetische Probe auf Fragmente von Nukleinsäure mit einer mittleren Fragmentgröße reduziert wird, die dem spezifizierten Zeitintervall entspricht, wobei die Eingangswellenform Tonbursts mit ausgewählter Wiederholungsrate und relativer Einschaltdauer umfasst.

12. Vorrichtung nach Anspruch 11, wobei das spezifizierte Zeitintervall mit einer Zielfragmentgröße korreliert ist, die sich der mittleren Fragmentgröße nähert, wenn die Eingangswellenform an den Wandler (102) angelegt wird, um über das spezifizierte Zeitintervall akustische Energie zu der genetischen Probe zu leiten.

13. Vorrichtung nach Anspruch 11, wobei der Funkfrequenzgenerator (109) ferner konfiguriert ist, um:
das spezifizierte Zeitintervall als eine Funktion eines Spannungspegels für die Eingangswellenform und einer Zielfragmentgröße für die mittlere Fragmentgröße zu schätzen; oder
das spezifizierte Zeitintervall als eine Funktion einer Zielfragmentgröße für die mittlere Fragmentgröße und der Wiederholungsrate der Tonbursts für die Eingangswellenform zu schätzen; oder
das spezifizierte Zeitintervall als eine Funktion einer Zielfragmentgröße für die mittlere Fragmentgröße und der mittleren Einschaltdauer der Tonbursts für die Eingangswellenform zu schätzen; oder
das spezifizierte Zeitintervall als eine Funktion einer Zielfragmentgröße für die mittlere Fragmentgröße und einer RF-Frequenz der Eingangswellenform zu schätzen; oder
das spezifizierte Zeitintervall als eine Funktion einer Zielfragmentgröße für die mittlere Fragmentgröße und einer Phasenkombination der Tonbursts für die Eingangswellenform an Komponentenelementen der FASA-Konfiguration zu schätzen.

14. Vorrichtung nach Anspruch 11, wobei der Funkfrequenzgenerator (109) ferner konfiguriert ist, um die Tonbursts mit einer ausgewählten Form zu verwenden.

15. Vorrichtung nach Anspruch 11, wobei der Funkfrequenzgenerator (109) ferner konfiguriert ist, um eine Dauer und Amplitude der Tonbursts systematisch einzustellen.

## Revendications

1. Procédé d'utilisation de rayonnement ultrasonique pour commander la fragmentation d'un échantillon génétique qui inclut des chaînes d'acides nucléiques, le procédé comprenant :
fournir un transducteur (102) qui inclut un substrat (104) avec des électrodes supérieure et inférieure (106, 108) configuré comme un actionneur de Fresnel à secteur annulaire (FASA) qui dirige de l'énergie acoustique dans une direction cible ;
fournir un récipient d'échantillon (110) pour contenir l'échantillon génétique, le récipient d'échantillon (110) étant disposé dans la direction cible du transducteur (102) et le récipient d'échantillon (110) étant couplé acoustiquement au transducteur (102) à travers un milieu de couplage (116) ; et
appliquer une forme d'onde d'entrée au transducteur (102) pour diriger de l'énergie acoustique vers l'échantillon génétique sur un intervalle de temps spécifié, l'échantillon génétique étant réduit à des fragments d'acide nucléique ayant une taille de fragment moyenne qui correspond à l'intervalle de temps spécifié, dans lequel la forme d'onde d'entrée comprend des impulsions sonores ayant une cadence de répétition et un facteur d'utilisation sélectionnés.

2. Procédé selon la revendication 1, dans lequel le substrat (104) inclut un matériau piézoélectrique et les électrodes (106, 108) incluent un matériau électriquement conducteur.

3. Procédé selon la revendication 1, dans lequel l'échantillon génétique inclut au moins un élément parmi de l'ADN (acide désoxyribonucléique), de l'ARN (acide ribonucléique) ou des cellules lysées.

4. Procédé selon la revendication 1, dans lequel la forme d'onde d'entrée inclut une fréquence qui est approximativement une fréquence résonante du substrat (104).

5. Procédé selon la revendication 1, dans lequel l'intervalle de temps spécifié est en corrélation avec une taille de fragment cible qui approche la taille de fragment moyenne lorsque la forme d'onde d'entrée est appliquée au transducteur (102) pour diriger de l'énergie acoustique vers l'échantillon génétique sur l'intervalle de temps spécifié.

6. Procédé selon la revendication 1, comprenant en outre :
estimer l'intervalle de temps spécifié comme une fonction d'un niveau de tension pour la forme d'onde d'entrée et d'une taille de fragment cible pour la taille de fragment moyenne ; ou
estimer l'intervalle de temps spécifié comme une fonction d'une taille de fragment cible pour la taille de fragment moyenne et de la cadence de répétition des impulsions sonores pour la forme d'onde d'entrée ; ou
estimer l'intervalle de temps spécifié comme une fonction d'une taille de fragment cible pour la taille de fragment moyenne et du facteur d'utilisateur des impulsions sonores pour la forme d'onde d'entrée ; ou
estimer l'intervalle de temps spécifié comme une fonction d'une taille de fragment cible pour la taille de fragment moyenne et d'une fréquence RF de la forme d'onde d'entrée ; ou
estimer l'intervalle de temps spécifié comme une fonction d'une taille de fragment cible pour la taille de fragment moyenne et d'une combinaison de phases des impulsions sonores pour la forme d'onde d'entrée au niveau d'éléments de composant de la configuration FASA.

7. Procédé selon la revendication 1, dans lequel les électrodes supérieure et inférieure (106, 108) incluent chacune une pluralité de segments annulaires qui recouvrent un secteur angulaire compris entre 0 et 360 degrés.

8. Procédé selon la revendication 1, dans lequel les électrodes supérieure et inférieure (106, 108) incluent chacune une électrode composite qui inclut une pluralité d'éléments de secteur qui divisent une zone en secteurs approximativement égaux, chaque élément de secteur incluant une pluralité de segments annulaires qui recouvrent chacun un secteur angulaire correspondant.

9. Procédé selon la revendication 1, dans lequel les impulsions sonores ayant une forme sélectionnée.

10. Procédé selon la revendication 1, dans lequel une durée et une amplitude des impulsions sonores sont ajustées systématiquement.

11. Appareil pour utiliser un rayonnement ultrasonique pour commander la fragmentation d'un échantillon génétique qui inclut des chaînes d'acides nucléiques, l'appareil comprenant :
un transducteur (102) qui inclut un substrat (104) avec des électrodes supérieure et inférieure (106, 108) configuré comme un actionneur de Fresnel à secteur annulaire (FASA) qui dirige de l'énergie acoustique dans une direction cible ;
un récipient d'échantillon (110) configuré pour contenir l'échantillon génétique, le récipient d'échantillon (110) étant disposé dans la direction cible du transducteur (102) et le récipient d'échantillon (110) étant couplé acoustiquement au transducteur (102) à travers un milieu de couplage (116) ; et
un générateur de radiofréquence (109) configuré pour appliquer une forme d'onde d'entrée au transducteur (102) pour diriger de l'énergie acoustique vers l'échantillon génétique sur un intervalle de temps spécifié, l'échantillon génétique étant réduit à des fragments d'acide nucléique ayant une taille de fragment moyenne qui correspond à l'intervalle de temps spécifié, dans lequel la forme d'onde d'entrée comprend des impulsions sonores ayant une cadence de répétition et un facteur d'utilisation sélectionnés.

12. Appareil selon la revendication 11, dans lequel l'intervalle de temps spécifié est en corrélation avec une taille de fragment cible qui approche la taille de fragment moyenne lorsque la forme d'onde d'entrée est appliquée au transducteur (102) pour diriger de l'énergie acoustique vers l'échantillon génétique sur l'intervalle de temps spécifié.

13. Appareil selon la revendication 11, dans lequel le générateur de radiofréquence (109) est en outre configuré pour :
estimer l'intervalle de temps spécifié comme une fonction d'un niveau de tension pour la forme d'onde d'entrée et d'une taille de fragment cible pour la taille de fragment moyenne ; ou
estimer l'intervalle de temps spécifié comme une fonction d'une taille de fragment cible pour la taille de fragment moyenne et de la cadence de répétition des impulsions sonores pour la forme d'onde d'entrée ; ou
estimer l'intervalle de temps spécifié comme une fonction d'une taille de fragment cible pour la taille de fragment moyenne et du facteur d'utilisateur des impulsions sonores pour la forme d'onde d'entrée ; ou
estimer l'intervalle de temps spécifié comme une fonction d'une taille de fragment cible pour la taille de fragment moyenne et d'une fréquence RF de la forme d'onde d'entrée ; ou
estimer l'intervalle de temps spécifié comme une fonction d'une taille de fragment cible pour la taille de fragment moyenne et d'une combinaison de phases des impulsions sonores pour la forme d'onde d'entrée au niveau d'éléments de composant de la configuration FASA.

14. Appareil selon la revendication 11, dans lequel le générateur de radiofréquence (109) est en outre configuré pour utiliser les impulsions sonores ayant une forme sélectionnée.

15. Appareil selon la revendication 11, dans lequel le générateur de radiofréquence (109) est en outre configuré pour ajuster une durée et une amplitude des impulsions sonores systématiquement.
